Europäisches Patentamt

(19) European Patent Office

Office européen des brevets

(11) Publication number: **0 253 717 B1**

(12) # EUROPEAN PATENT SPECIFICATION

(45) Date of publication of patent specification :.
20.02.91 Bulletin 91/08

(51) Int. Cl.$^5$: **A61K 9/06**, A61K 31/415,
A61K 31/535

(21) Application number : 87401593.6

(22) Date of filing : 07.07.87

(54) Combinations of beta-blockers and pilocarpine.

(30) Priority : 09.07.86 FR 8610011

(43) Date of publication of application :
20.01.88 Bulletin 88/03

(45) Publication of the grant of the patent :
20.02.91 Bulletin 91/08

(84) Designated Contracting States :
AT BE CH DE ES FR GB GR IT LI LU NL SE

(56) References cited :
US-A- 4 474 751
CHEMICAL ABSTRACTS,vol. 99, 1983, page
335, ref.no. 27939u; Columbus, Ohio, US M.
SANTUS et al.:"Biopharmacy and formulation
of eye drops containing pilocarpine with
propanolol."

(73) Proprietor : LABORATOIRES MERCK, SHARP
& DOHME-CHIBRET
3, Avenue Hoche
F-75008 Paris (FR)

(72) Inventor : Sliwa, Jean
11, avenue Pierre Curie
F-63400 Chamalieres (FR)
Inventor : Py, Daniel
22, Ferncliff Terrace
Short Hills New Jersey (US)

(74) Representative : Warcoin, Jacques et al
Cabinet Régimbeau 26, avenue Kléber
F-75116 Paris (FR)

EP 0 253 717 B1

## Description

### SUMMARY OF THE INVENTION

This invention is concerned with an ophthalmological formulation comprising in combination a β-adrenergic receptor antagonist and pilocarpine as active ingredients and the use of that formulation in a twice a day dosage regimen for the treatment of elevated intraocular pressure in patients refractory to treatment with β-adrenergic receptor antagonists alone.

It is also concerned with preformulation products comprising either a solid and a liquid or two liquids which are combined shortly before use, either by the pharmacist or the patient, and means for combining the two components of the preformulation products.

### BACKGROUND

US 4 474 751 relates to an ophthalmic drug delivery system ready to use utilising thermosetting gels consisting in polymers.

### BACKGROUND OF THE INVENTION

Since the introduction in 1979 of timolol eye drops as a twice daily treatment for glaucoma, it has been the experience of many clinicians that in between 10% and 32% of patients the intraocular pressure (IOP) is not adequately controlled even with 0.5% timolol twice daily. In over half of these it is found possible to gain control by adding pilocarpine to the regimen. In time, probably due to progression of the disease process a few more experience loss of IOP control and require additional therapy.

It has been reported that combining timolol with other therapies can be partially additive. Indeed it could be viewed as rational to combine timolol, which lowers IOP by reducing aqueous production with a drug like pilocarpine acting by enhancing outflow through the trabecular meshwork. Pilocarpine has a 6-8 hour period of efficacy and is normally given four times a day for that reason. Even so it has been shown that the diurnal curve of IOP on pilocarpine therapy can show swings of pressure of 5 mmHg or more. Thus it would be expected that additional IOP lowering achieved by adding pilocarpine to timolol would not be smooth unless the pilocarpine was administered 6 hourly thus losing the benefit of twice a day dosage.

Now, with the present invention, there is provided a combination therapy for elevated intraocular pressure and glaucoma comprising twice a day topical ocular administration of a ß-blocker and pilocarpine which results in a smooth, well controlled reduced intraocular pressure.

Pilocarpine is chemically unstable at pH values of about 4 and greater, but ophthalmic formulations of that low a pH are not acceptable to patients because of the stinging sensation on instillation in the eye. Accordingly, formulations of pilocarpine such as those of this invention to gain patient acceptance should have a pH of about 5.5 to 7.0 and are best if constituted by the retail pharmacist at the time of dispensing the prescription or by the patient just before the first administration.

With this invention there are provided preformulation products comprising a solid and a solution or two separate solutions.

In addition to patient acceptance, it is also important that an actual combination be used as opposed to administration of each component separately inasmuch as administration of drops of a second component has a tendency to wash away the drops of the first component thus eliminating the benefits to be derived therefrom. Furthermore double administrations tend to reduce patient compliance.

### DETAILED DESCRIPTION

The novel method of treatment of this invention comprises the topical ocular administration, twice a day, of one or two drops of a novel formulation comprising as active ingredients an ophthalmic β-blocker and pilocarpine, wherein the concentration of the β-blocker i 0.5 to 1.0% (w/v) and the concentration of the pilocarpine is 2% to 4% (w/v).

The β-blockers useful in the novel method of this invention include [carteolol, befunolol, metipranolol, pindolol, betaxolol, levobunolol, and timolol],and ophthalmologically acceptable salts thereof.

By far the most preferred β-blocker as an ophthalmic agent, and in the present invention is timolol maleate.

The novel preformulation products of this invention to permit constitution just prior to use to form the novel formulation of this invention comprise a sterile solid and a sterile solution, or two sterile solutions.

In the case of the solid/liquid preformulation, the solid is sterile lyophilized pilocarpine and the liquid is a sterile buffered solution of timolol maleate. In the case of a liquid/liquid preformulation one liquid is a sterile aqueous solution of pilocarpine and a β-blocker ; and the other a sterile aqueous solution of buffers. In each case the components have adequate shelf life, with means for combining the two solutions in a sterile fashion to provide a combination formulation of pH 6.0 to 6.8 so that the tendency to sting on administration is minimized.

A delivery system for use with the preformulations requiring reconstitution before use is provided by U.S. Patent 4,573,506.

Typical formulations are as follows :

## Wet/Wet Formulations – Borate Buffer

|  | C-TP2 mg/ml | C-TP4 mg/ml | A-TP2 mg/ml | A-TP4 mg/ml |
|---|---|---|---|---|
| **Concentrate** | | | | |
| Timolol maleate | 13.66 | 13.66 | 13.66 | 13..66 |
| Pilocarpine HCl | 40.00 | 80.00 | 40.00 | 80.00 |
| Benzalkonium Chloride | 0.11 | 0.11 | 0.11 | 0.11 |
| Water for Injection | q.s. | q.s. | q.s. | q.s. |
| **Diluent** | | | | |
| Boric Acid | 8.00 | 12.00 | 8.00 | 25.00 |
| Sodium Borate | 7.00 | 30.00 | 14.00 | 8.00 |
| Benzalkonium Chloride | 0.11 | 0.11 | 0.11 | 0.11 |
| Water for Injection | q.s. | q.s. | q.s. | q.s. |
| **Reconstituted Solution** | | | | |
| Timolol Maleate | 6.83 | 6.83 | 6.83 | 6.83 |
| Pilocarpine HCl | 20.00 | 40.00 | 20.00 | 40.00 |
| Boric Acid | 4.00 | 6.00 | 4.00 | 4.00 |
| Sodium Borate | 3.50 | 15.00 | 7.00 | 12.50 |
| Benzalkonium Chloride | 0.11 | 0.11 | 0.11 | 0.11 |
| Water for Injection | q.s. | q.s. | q.s. | q.s. |
|  | pH = 6.3-6.0 | pH = 6.3-6.0 | pH = 6.8-6.5 | pH = 6.8-6.5 |

Wet/Wet Formulations — Phosphate Buffer

|  | B-TP2 mg/ml | B-TP4 mg/ml | TP2 mg/ml | TP4 mg/ml |
|---|---|---|---|---|
| **Concentrate** |  |  |  |  |
| Timolol maleate | 13.66 | 13.66 | 13.66 | 13.66 |
| Pilocarpine HCl | 40.00 | 80.00 | 40.00 | 80.00 |
| Benzalkonium Chloride | 0.11 | 0.11 | 0.11 | 0.11 |
| Water for Injection | q.s. | q.s. | q.s. | q.s. |
| **Diluent** |  |  |  |  |
| Dibasic Sodium Phosphate, $12H_2O$ | 22.60 | 45.50 | 39.40 | 80.00 |
| Sodium Dihydrogen Phosphate, $2H_2O$ | 8.70 | 23.70 | 1.00 | – |
| Benzalkonium Chloride | 0.11 | 0.11 | 0.11 | 0.11 |
| Water for Injection | q.s. | q.s. | q.s. | q.s. |
| **Reconstituted Solution** |  |  |  |  |
| Timolol Maleate | 6.83 | 6.83 | 6.83 | 6.83 |
| Pilocarpine HCl | 20.00 | 40.00 | 20.00 | 40.00 |
| Dibasic Sodium Phosphate, $12H_2O$ | 11.30 | 25.25 | 19.70 | 40.00 |
| Sodium Dihydrogen Phosphate, $2H_2O$ | 4.35 | 11.85 | 0.50 | – |
| Benzalkonium Chloride | 0.11 | 0.11 | 0.11 | 0.11 |
| Water for Injection | q.s. | q.s. | q.s. | q.s. |
|  | pH = 6.3-6.0 | pH = 6.3-6.0 | pH = 6.8-6.5 | pH = 6.8-6.5 |

## Wet/Dry Formulations - Borate Buffer
### pH of the reconstituted solution: 6.8 - 6.5

|  | M-TP2 | M-TP4 |
|---|---|---|
| **Lyophilisate** | | |
| Pilocarpine HCl (for 5 ml diluent) | 100 mg | 200 mg |
| **Diluent** | mg/ml | mg/ml |
| Timolol Maleate | 6.83 | 6.83 |
| Sodium Borate | 4.00 | 12.50 |
| Boric Acid | 7.00 | 4.00 |
| Benzalkonium Chloride | 0.11 | 0.11 |
| Water for Injection | q.s. | q.s. |
| **Reconstituted Solution** | mg/ml | mg/ml |
| Timolol Maleate | 6.83 | 6.83 |
| Pilocarpine HCl | 20.00 | 40.00 |
| Sodium Borate | 4.00 | 12.50 |
| Boric Acid | 7.00 | 4.00 |
| Benzalkonium Chloride | 0.11 | 0.11 |
| Water for Injection | q.s. | q.s. |

## Wet/Dry Formulations – Phosphate Buffer
### pH of the reconstituted solution:  6.8 – 6.5

| | L-TP2 | L-TP4 |
|---|---|---|
| **Lyophilisate** | | |
| Pilocarpine HCl (for 5 ml diluent) | 100 mg | 200 mg |
| **Diluent** | mg/ml | mg/ml |
| Timolol Maleate | 6.83 | 6.83 |
| Dibasic Sodium Phosphate, $12H_2O$ | 19.70 | 40.00 |
| Sodium Dihydrogene Phosphate, $2H_2O$ | 0.50 | – |
| Benzalkonium Chloride | 0.11 | 0.11 |
| Water for Injection | q.s. | q.s. |
| **Reconstituted Solution** | mg/ml | mg/ml |
| Timolol Maleate | 6.83 | 6.83 |
| Pilocarpine HCl | 20.00 | 40.00 |
| Dibasic Sodium Phosphate, $12H_2O$ | 19.70 | 40.00 |
| Sodium Dihydrogene Phosphate, $2H_2O$ | 0.50 | – |
| Benzalkonium Chloride | 0.11 | 0.11 |
| Water for Injection | q.s. | q.s. |

The study leading to the present invention was designed to establish, by recording full 24 hour diurnal IOP curves, whether pilocarpine used twice daily with timolol achieved lower IOP than timolol alone and whether the control was smooth. We further studied whether using pilocarpine 6 hourly with timolol 12 hourly produced smoother or more effective control of IOP than when both were administered 12 hourly.

## PATIENTS AND METHOD

Patients aged 40 or over, male or female, all white, with either ocular hypertension or primary open angle glaucoma with an IOP in one or both eyes of 22 mmHg or more at one time point each day while receiving timolol 0.5% twice a day (bd) alone were admitted. Patients had been on timolol 0.5% bd, either alone or in combination for at least six weeks prior to study entry and had been on timolol 0.5% bd as their sole glaucoma therapy for at least two weeks prior to study admission.

Secondary glaucoma was an exclusion as was a history of glaucoma surgery or laser trabeculoplasty/gonioplasty. Patients for whom timolol was contraindicated by the datasheet were excluded and also excluded were those on a concurrent ß-blocker, carbonic anhydrase inhibitor, or clonidine. 24 patients entered the study in the order in which they appeared in the Out Patient Department for their routine appointments. There were 11 males and 13 females with a modal age of 70 years (range 45-81 years).

Procedure

1. All patients had their central visual fields plotted by Goldman Perimetry prior to study entry.

2. Patients were admitted for a 24 hour diurnal curve (i.e. : IOP recorded at 06.00, 10.00, 14.00, 18.00, 24.00 hours approximately, the 06.00 and 18.00 recordings were immediately prior to the instillation of the drops). All pressures were measured by the same observer using the same Goldman applanation tonometer.

3. Following recording of the baseline Diurnal Curve on timolol 0.5% bd patients were instructed to add pilocarpine 2% bd to each eye administered 5 minutes after the timolol and to return in 2 weeks for a second Diurnal Curve.

4. If the IOP was still >21 mmHg at one or more time points in either eye during the second Diurnal Curve the patient was given pilocarpine 4% bd (to replace the pilocarpine 2%) and told to return in a further 2 weeks for a further Diurnal Curve.

5. Finally all patients were given timolol 0.5% bd plus pilocarpine 2% or 4% four times a day (qid) depending on the result of the previous dose titration and told to return in a further 2 weeks for their final Diurnal Curve.

Overall it was apparent that the major effect of adding pilocarpine bd to timolol was that an additional significant reduction in IOP occurred. Patients who were previously uncontrolled at some time point were now brought under control as defined by an IOP of 21 mmHg. An additional benefit of adding pilocarpine bd to timolol was that the range of IOP variations over a 26.5 hour period could be reduced although the number of patients in whom the range would be reduced to <5 mmHg was of equivocal significance.

Taking these facts together it was possible to draw clinical reassurance that if pilocarpine bd were used with timolol bd there would not be periods during the 24 hours when the pilocarpine would be failing to produce an additional and adequate pressure lowering. Comparing the smoothness of control found in this study with that found in previous studies it appears that timolol has a smoothing effect on the often considerable diurnal variation seen in eyes treated with pilocarpine.

The nature of the synergistic mechanism between timolol and pilocarpine appears to have at least a 12 hour period of action. Purely lowering the intraocular pressure more may reduce the variation. It may be that a truly synergistic effect is being seen and that even a small increase in outflow is sufficient to lower the IOP in the presence of a suppressed aqueous production.

No clear cut advantage could be seen from the use of pilocarpine qid as opposed to bd when coadministered with timolol 0.5% bd. The more frequent pilocarpine administration did not greatly influence the flatness of the IOP/Time plot which is where a difference would have been intuitively expected. A combination of timolol 0.5% with either pilocarpine 2% or 4% administered twice daily is a useful addition to the agents available for glaucoma management.

**Claims**

1. An ophthalmic preformulation product comprising lyophilized pilocarpine hydrochloride and a solution of an ophthalmic β-blocker and a buffer and means for combining the two to produce a solution comprising 0.5 to 1% (w/v) of the β-blocker and 2 to 4% of pilocarpine with pH 6.0 to 6.8.

2. The preformulation product of claim 1 wherein the β-blocker is timolol maleate and the buffer is a borate or phosphate buffer.

3. The preformulation of anyone of claims 1 to 2 wherein the buffer is a phosphate buffer.

4. A pharmaceutical composition for treating elevated intraocular pressure and glaucoma comprising a ophthalmic formulation reconstituted with the preformulation product of claims 1 to 3.

5. The use of pilocarpine hydrochloride, an ophthalmic β-blocker and a buffer to prepare the preformulation product of any one of claims 1 to 3 useful in the treatment of elevated intraocular pressure and glaucoma.

**Ansprüche**

1. Ophthalmisches Vorformulierungsprodukt, welches gefriergetrocknetes Pilocarpinhydrochlorid und eine Lösung eines ophthalmologischen β-Blockers und einen Puffer sowie Mittel zur Kombination der beiden zur Erzeugung einer Lösung mit 0,5 bis 1% (G/V) des β-Blockers und 2 bis 4% Pilocarpin bei pH 6,0 bis 6,8 umfaßt.

2. Vorformulierungsprodukt nach Anspruch 1, worin der β-Blocker Timololmaleat ist und der Puffer ein Boratoder phosphatpuffer.

3. Vorformulierung nach einem der Ansprüche 1 bis 2, worin der Puffer ein Phosphatpuffer ist.

4. Pharmazeutische Zusammensetzung zur Behandlung von erhöhtem Augeninnendruck und Glaucomen, welche eine aus dem Vorformulierungsprodukt der Ansprüche 1 bis 3 neugebildete ophthalmische Formulierung umfaßt.

5. Verwendung von Pilocarpinhydrochlorid, einem ophthalmischen β-Blocker und einem Puffer zur Herstellung des zur Behandlung von erhöhtem Augeninnendruck und Glaucomen geeigneten Vorformulierungsprodukts nach einem der Ansprüche 1 bis 3.

**Revendications**

1. Produit ophtalmique en préformulation qui comprend du chlorhydrate de pilocarpine lyophilisé et une solution d'un bêta-bloquant ophtalmique et d'un tampon et des moyens pour combiner les deux afin de produire une solution comprenant 0,5 à 1% (poids/vol) du bêta-bloquant et 2 à 4 % de pilocarpine avec un pH de 6,0 à 6,8.

2. Produit en préformulation selon la revendication 1, dans lequel le bêta-bloquant est le maléate de timolol et le tampon est un tampon de borate ou de phosphate.

3. Préformulation selon l'une quelconque des revendications 1 ou 2, dans laquelle le tampon est un tampon de phosphate.

4. Composition pharmaceutique pour le traitement d'une tension intra-oculaire élevée et du glaucome qui comprend une formulation ophtalmique reconstituée avec le produit en préformulation selon les revendications 1 à 3.

5. Utilisation du chlorhydrate de pilocarpine, d'un bêta-bloquant ophtalmique et d'un tampon pour préparer le produit en préformulation selon l'une quelconque des revendications 1 à 3, utile dans le traitement du glaucome et de la tension intra-oculaire élevée.